# DEMANDE DE BREVET EUROPEEN

(11) **EP 1 570 841 A1**
(43) Date de publication de la demande: **07.09.2005**
(21) Numéro de dépôt: 05290440.6
(22) Date de dépôt: 25.02.2005
(51) Int. Cl.: A61K 7/48

(54) **Utilisation d'au moins un inhibiteur des canaux calciques pour prevenir et/ou traiter la peau grasse**

(30) Priorité: 01.03.2004 FR 0450395
(71) Demandeur: L'OREAL, 75008 Paris (FR)
(72) Inventeur: Rubinstenn, Gilles, 75005 Paris (FR); Breton, Lionel, 78000 Versailles (FR)
(74) Mandataire: Bernstein, Claire Jacqueline

(57) **Abrégé**

L'invention se rapporte à l'utilisation d'un antagoniste des canaux calciques, notamment un sel de manganèse, destiné à limiter la lipogénèse sébacée, par exemple, pour le traitement de la peau grasse hyper-séborrhéique.

## Description

L'invention se rapporte à l'utilisation d'un antagoniste des canaux calciques, notamment un sel de manganèse, destiné à diminuer la lipogénèse sébacée, par exemple, pour le traitement de la peau grasse hyper-séborrhéique.

Une peau grasse hyper-séborrhéique est caractérisée par une sécrétion et une excrétion exagérées de sébum. Classiquement, un taux de sébum supérieur à 200 µg/cm² est considéré comme caractéristique d'une peau grasse.
Une peau grasse est une peau d'aspect luisant, épaisse et dont les orifices folliculaires sont dilatés ou comblés de minuscules spicules cornés, voire par des comédons (résultant cependant davantage d'un phénomène de rétention que d'une augmentation de l'excrétion). La peau grasse est généralement associée à un défaut de desquamation et à un grain de peau épais. Un autre inconvénient des peaux grasses est la mauvaise tenue du maquillage ce qui implique de traiter la peau pour limiter le flux de sébum avant d'appliquer le maquillage.

En plus de ces désordres esthétiques, l'excès de sébum peut servir de support au développement anarchique de flore bactérienne saprophyte (en particulier, *Propionibacterium acnes* et *Pityrosporum ovale*) et causer des comédons et/ou des lésions acnéiques.
Toutefois, l'acné et la peau grasse ne vont pas systématiquement de paire. En effet, si l'acné présente une occurrence importante dans les cas d'hyperséborrhée androgénique (typiquement dans le cas des acnés juvéniles), elle peut se développer chez des individus dont la production de sébum n'est pas particulièrement élevée. Inversement des individus à peau grasse ne développent pas systématiquement de lésion acnéique.

Le sébum est le produit naturel de la glande sébacée qui constitue une annexe de l'unité pilo-sébacée. Il s'agit essentiellement d'un mélange plus ou moins complexe de lipides comprenant du squalène, des triglycérides, des cires aliphatiques, des cires de cholestérol et, éventuellement, du cholestérol libre (Stewart, 1992). L'action des lipases bactériennes convertit une part variable de triglycérides formés en acides gras libres.

Le sébocyte constitue la cellule compétente de la glande sébacée. La production de sébum est associée à un programme de différentiation terminale de cette cellule.

Durant cette différentiation, l'activité métabolique du sébocyte est essentiellement axée sur la biosynthèse des lipides (la lipogénèse) et, plus précisément, sur la néosynthèse d'acides gras.

Il existe donc un besoin pour une composition cosmétique ou dermatologique permettant d'éviter les troubles esthétiques et le développement de lésions acnéiques associés à la surproduction de sébum.

Plusieurs compositions cosmétiques susceptibles d'offrir une solution à ces problèmes ont été proposées dans l'art antérieur. Ces compositions comprennent notamment à titre de principe actif :
- des poudres qui permettent d'absorber le sébum et donc de matifier la peau par un effet mécanique ;
- des alpha- ou béta-hydroxyacides qui, de par leur effet kératolytique, favorisent l'expulsion des bouchons cornés ;
- des actifs astringents permettant de lutter contre la dilatation des follicules sébacés ;
- des actifs exfoliants permettant de diminuer l'épaisseur de la peau ; et
- des actifs qui agissent sur les mécanismes biochimiques, notamment les enzymes, impliquées dans la production de sébum.

Toutefois, l'efficacité de ces compostions est relative car la plupart ne limite pas la production sébacée mais ne font qu'absorber le sébum.

Le but de la présente invention est justement de fournir des moyens pour traiter le problème des peaux grasses à la base, en contrôlant la lipogénèse, plutôt qu'en traitant *a posteriori* les conséquences d'une lipogénèse excessive.

Dans le cadre de la recherche de composés susceptibles d'avoir des propriétés sébo-régulatrices, la Demanderesse a mis en évidence que les antagonistes des canaux calciques peuvent inhiber de façon significative la lipogénèse.

Pour qu'une substance soit reconnue comme un inhibiteur des canaux calciques, autrement appelé dans le texte antagoniste des canaux calciques, elle doit pouvoir diminuer la concentration intracellulaire en calcium ou diminuer la liaison du calcium aux protéines intracellulaires, comme par exemple la calmoduline (Galizzi J.P. et al. J. Biol. Chem. 1987, 262, p.6947 - Okamiya Y. et al. Eur. J. Pharmacol. 1991, 205, p.49 - Wagner J.A. et al. J. Neurosci. 1988, 8, p.3354 - Lee H.R. et al., Life Sci. 1984, 35 p.721 - Shoemaker H. & Lauger S. Eur. J. Pharmacol. 1985, 111 p.273 - Reynolds et al. J. Pharmacol. Exp. Ther. 1986, 237 p. 731).

Ainsi l'objet de la présente invention se rapporte à l'utilisation cosmétique d'au moins un antagoniste des canaux calciques pour diminuer la lipogénèse sébacée.

En particulier, l'utilisation selon la présente invention a pour but de prévenir et/ou corriger les altérations cutanées provoquées et/ou associées à une hyper-sécrétion sébacée, telles que l'aspect luisant de la peau et/ou un grain de peau épais et/ou des orifices folliculaires dilatés. Elle a aussi pour but de prévenir et/ou traiter les peaux grasses.

L'antagoniste des canaux calciques pourra être choisi parmi les composés suivants : chlorpromazine, verapamil, nifedipine, prenylamine, nisoldipine.

L'antagoniste des canaux calciques pourra également être du manganèse, en effet, le manganèse a été décrit comme étant un inhibiteur des canaux calciques notamment par Suzuki et al. (1982), il pourra alors se présenter sous forme ionique ou sous forme de sels minéraux ou organiques isolés et/ou présents dans des extraits naturels, végétaux, tels que des extraits de noix (*Juglans regia*) ou bactériens.

Les sels organiques de manganèse utilisables selon l'invention pourront être un sel de manganèse d'un acide carboxylique tel que le gluconate de manganèse, le carbonate de manganèse, l'acétate de manganèse, le citrate de manganèse, l'oléate de manganèse, l'oxalate de manganèse, le linéolate de manganèse ou le linolénate de manganèse, ou un sel de manganèse d'un acide aliphatique dicarboxylique tel que l'acide sébacique, l'acide séborique ou l'acide azélaique.

Les sels inorganiques de manganèse pourront être choisis parmi les sels minéraux tels que le chlorure de manganèse, le borate de manganèse, le nitrate de manganèse, le phosphate de manganèse ou le sulfate de manganèse.

Les oligoéléments sont des composants de l'organisme, d'origine minérale. Ils sont présents en très petites quantités dans l'organisme, et même pour certains seulement à l'état de traces : ce sont notamment, le fer, le zinc, le fluor, le cuivre, l'iode, le manganèse, le cobalt, le sélénium, le vanadium, le molybdène, le chrome.
On regroupe sous le terme d'oligoéléments une vingtaine d'éléments présents à l'état de traces dans l'organisme. Tous ne peuvent pas être qualifiés d'essentiels dans la mesure où l'on ne possède pas encore les preuves expérimentales de la nécessité vitale de leur présence.

Les rôles joués par ces oligoéléments sont variables et divers :
- constitution des tissus ;
- régulateur des mouvements d'eau ;
- rôle dans l'excitabilité neuromusculaire ;
- élaboration des hormones, des enzymes, etc... soit par incorporation directe, soit par un mécanisme catalytique ;
- les glucides, les lipides, les protéines et les vitamines ne peuvent être apportés au corps humain que si celui-ci dispose de suffisamment de sels minéraux.

Par exemple, le calcium et le phosphore servent à fabriquer la matière vivante et entrent dans sa composition.
Le cuivre ou le chrome sont utilisés comme accélérateurs de réactions enzymatiques (catalyseurs). Leur rôle est essentiel dans la fabrication des protéines, le transport de l'oxygène et dans la régulation de le taux de sucre par l'insuline.

Le rein élimine quotidiennement des oligoéléments, aussi, notre alimentation doit en apporter chaque jour des quantités suffisantes.

Les oligoéléments sont le plus souvent apportés par les aliments d'origine végétale.

A titre d'exemple, les principaux oligoéléments sont :
- les sels de calcium qu'on trouvent dans le lait, le fromage, les végétaux frais ;
- le fer qui est essentiel dans la composition des globules rouges se trouve dans le persil, le foie de veau, les légumes secs ;
- le phosphore qui est utilisé dans la formation des cellules nerveuses du cerveau se retrouve, par exemple, dans la viande, le poisson, les oeufs.

Le manganèse est un métal très répandu à la surface de la croûte terrestre. Il appartient au groupe VIIa de Mendeleïev, son numéro atomique est 25, son poids atomique 54,93. Le manganèse présente plusieurs valences (1 à 7), les formes di- et trivalentes sont celles qui sont biologiquement les plus actives.

Le rôle biologique du manganèse est très important et, même si les effets néfastes d'une carence n'ont pas été constatés de façon irréfutable chez l'homme, les conséquences des déficits examinés chez l'animal montrent que ce métal est impliqué dans de nombreux métabolismes.

Le manganèse est impliqué dans le métabolisme de :
- la coagulation,
- la thermogénèse (par son action sur le système thyroïdien),
- l'immunité, où le manganèse semble être nécessaire à une synthèse correcte des anticorps (le manganèse a une action inhibitrice sur la stimulation des lymphocytes B et T s'il est ajouté au milieu très peu de temps après un agent mitogène),
- la reproduction, sa carence entraînant une baisse de fertilité des femelles et des mâles.

Deux modes d'action permettent d'expliquer une partie du rôle biologique du manganèse :
- l'activation de nombreuses enzymes : le manganèse est un métal qui active de nombreuses enzymes et lectines. Il intervient soit comme un élément dissociable, soit en faisant partie intégrante de la structure de l'enzyme (métalloprotéine) ;
- son activité inhibitrice vis-à-vis des canaux calciques : en effet, le manganèse est classiquement décrit comme un agent inhibiteur de la pénétration du calcium dans les cellules à activité sécrétrice (cellules de Langerhans pancréatiques) ou à activité électrique (myofibroblastes, neurones).

Chez l'homme, le manganèse ne se trouve qu'à l'état de trace. Par contre, il est présent dans le règne végétal, notamment dans les graines (noix par exemple), les fruits et certains légumes.

Le noyer (*Juglans regia*) de la famille des juglandacées, est un arbre cultivé en France (Périgord, Dauphiné) pour la production des noix. Le fruit est une drupe à exocarpe vert noircissant par oxydation à maturité (le brou), l'endocarpe dur, bivalvé, enveloppe deux cotylédons "cérébriformes" et volumineux.
Le principal constituant identifié est la juglone (5-hydroxy-1,4-naphtoquinone) qui existe dans la plante fraîche (feuille, brou) sous forme de glucoside du 1,4,5-trihydroxynaphtalène (2% dans le brou, 0,6% dans les feuilles) mais aussi sous forme libre, notamment dans la cire épicuticulaire. La juglone est accompagnée d'autres naphtoquinones (détectable par chromatographie en phase gazeuse) et de dérivés réduits. Les feuilles et péricarpes sont riches en tanins hydrolysables.
Les cotylédons de la graine sont utilisés dans l'alimentation et comme source d'huile : ils renferment en effet 50% voire plus d'une huile riche (60 à 70%) en acide linoléique.

Les extraits riches en manganèse sous forme solide ou liquide utilisables selon la présente invention proviennent plus particulièrement d'extrait aqueux ou des lyophilisats de ces mêmes extraits aqueux.

Dans le cadre de l'utilisation selon l'invention, l'antagoniste des canaux calciques est formulé dans une composition destinée à être administrée par voie orale ou topique.

Dans le cas d'une administration orale, la composition pourra se présenter sous forme de comprimés, de gélules, de dragées, de sirops, de suspensions, de solutions, de poudres, de granulés, d'émulsions, de suspensions de microsphères ou nanosphères ou vésicules lipidiques ou polymériques permettant une libération contrôlée.

De préférence, la composition se présente sous forme de complément nutritionnel comprenant un extrait de graines de Juglandacée, de préférence du genre *Juglans regia,* et/ou de manganèse minéral sous forme d'oligo-élément.

Selon un mode de réalisation particulier de l'invention, l'antagoniste des canaux calcique administré par voie oral est associé à au moins un actif complémentaire choisi parmi : les rétinoïdes, les caroténoïdes, la vitamine C et les agents antibactériens (en particulier, les phytoalexines).

Dans un autre mode de réalisation de l'invention, l'antagoniste des canaux calciques est formulé dans une composition destinée à être appliquée par voie topique.

Pour l'administration par voie topique, on préférera utiliser comme antagoniste des canaux calciques un sel de manganèse d'un acide aliphatique dicarboxylique, de préférence l'acide sébacique, l'acide séborique ou l'acide azélaique.

La quantité d'antagoniste des canaux calciques utilisable selon l'invention dépend bien évidemment de l'effet recherché et doit être en une quantité efficace pour favoriser la diminution de lipogénèse.

A titre d'exemple, l'antagoniste des canaux calciques pourra représenter :
- entre 10 et 1000 mg poids / kg de poids corporel / jour, de préférence 100 mg poids / kg de poids corporel / jour pour une administration par voie orale ;
- de 0,05 à 40 % du poids total de la composition et, préférentiellement, de 0,1 à 5 % en poids par rapport au poids total de la composition pour une administration par voie topique.

Lorsque la composition selon l'invention est destinée à être appliquée sur la peau, elle peut avoir la forme notamment d'une solution ou suspension aqueuse, alcoolique ou hydroalcoolique ou d'une suspension huileuse ou d'une solution ou d'une dispersion du type lotion ou sérum, d'une émulsion de consistance liquide ou semi-liquide du type lait, obtenues par dispersion d'une phase grasse dans une phase aqueuse (H/E) ou inversement (E/H), ou d'une suspension ou émulsion de consistance molle du type crème H/E ou E/H ou d'un gel aqueux ou anhydre, d'un onguent, d'une poudre libre ou compactée à utiliser telle quelle ou à incorporer dans un excipient, ou encore de microcapsules ou microparticules, ou de dispersions vésiculaires de type ionique et/ou non ionique. Ces compositions sont préparées selon les méthodes usuelles.

On peut également envisager une composition sous la forme d'une mousse ou encore sous forme de compositions pour aérosol comprenant également un agent propulseur sous pression.

Lorsque la composition est une émulsion, la proportion de la phase grasse peut aller de 2 à 80 % en poids, et de préférence de 5 à 50 % en poids par rapport au poids total de la composition. Les huiles, les cires, les émulsionnants et les coémulsionnants utilisés dans la composition sous forme d'émulsion sont choisis parmi ceux classiquement utilisés dans le domaine cosmétique. L'émulsionnant et le coémulsionnant sont présents, dans la composition, en une proportion allant de 0,1 à 30 % en poids, et de préférence de 0,5 à 20 % en poids par rapport au poids total de la composition. L'émulsion peut, en outre, contenir des vésicules lipidiques.

Lorsque la composition est une solution ou un gel huileux, la phase grasse peut représenter plus de 90 % du poids total de la composition.

De façon connue, la composition cosmétique peut contenir également des adjuvants habituels dans le domaine cosmétique, tels que les gélifiants hydrophiles ou lipophiles, les additifs hydrophiles ou lipophiles, les conservateurs, les antioxydants, les solvants, les parfums, les charges, les filtres, les absorbeurs d'odeur et les matières colorantes. Les quantités de ces différents adjuvants sont celles classiquement utilisées dans le domaine cosmétique, et par exemple de 0,01 à 10 % du poids total de la composition. Ces adjuvants, selon leur nature, peuvent être introduits dans la phase grasse, dans la phase aqueuse et/ou dans les sphérules lipidiques.

Comme huiles ou cires utilisables dans l'invention, on peut citer les huiles minérales (huile de vaseline), les huiles végétales (fraction liquide du beurre de karité, huile de tournesol), les huiles animales (perhydrosqualène), les huiles de synthèse (huile de Purcellin), les huiles ou cires siliconées (cyclométhicone) et les huiles fluorées (perfluoropolyéthers), les cires d'abeille, de carnauba ou paraffine. On peut ajouter à ces huiles des alcools gras et des acides gras (acide stéarique).

Comme émulsionnants utilisables dans l'invention, on peut citer par exemple le stéarate de glycérol, le polysorbate 60 et le mélange de PEG-6/PEG-32/Glycol Stéarate vendu sous la dénomination de Tefose^{R} 63 par la société Gattefosse.

Comme solvants utilisables dans l'invention, on peut citer les alcools inférieurs, notamment l'éthanol et l'isopropanol, le propylène glycol.

Comme gélifiants hydrophiles utilisables dans l'invention, on peut citer les polymères carboxyvinyliques (carbomer), les copolymères acryliques tels que les copolymères d'acrylates/alkylacrylates, les polyacrylamides, les polysaccharides tels que l'hydroxypropylcellulose, les gommes naturelles et les argiles, et, comme gélifiants lipophiles, on peut citer les argiles modifiées comme les bentones, les sels métalliques d'acides gras comme les stéarates d'aluminium et la silice hydrophobe, éthylcellulose, polyéthylène.

Selon une forme d'exécution avantageuse, la composition selon l'invention renferme en outre au moins un actif complémentaire choisi parmi :
- d'autres agents antiséborrhéiques tels que les oestrogènes, la cyprotérone et son acétate, les rétinoïdes et arorétinoïdes (l'acide 13 cis rétinoique), le rétinol et ses dérivés, le soufre et les dérivés soufrés, le peroxyde de benzoyle, les dérivés de zinc tels que le sulfate de zinc, le chlorure d'aluminium, le disulfure de sélénium, les vitamines B, et leurs mélanges ;
- des agents antimicrobiens et les phytoaléxines tel que le resvératrol, les antibiotiques, les antifongiques et leurs mélanges ;
- des agents antiseptiques (octopirox) ;
- des agents anti-inflammatoires et/ou anti-irritants tels que les acides ursolique et oléanolique et leurs sels, l'extrait de racine de paeonia suffruticosa, l'huile de callophyllum, l'acide 18 béta glycyrrhétinique et son sel de zinc, l'huile de tamanu, le laminaria saccharina extract ;
- des agents kératolytiques tels que l'acide salicylique et ses dérivés, les alpha-hydroxyacides, l'acide rétinoïque et ses dérivés, le rétinol et ses dérivés.

La quantité de ces actifs complémentaires peut varier dans une large mesure. Bien entendu, l'homme du métier veillera à choisir le ou les éventuels composés à ajouter aux compositions utilisables selon l'invention de telle manière que les propriétés avantageuses attachées intrinsèquement à la présente invention ne soient pas altérées par l'addition envisagée.

La présente invention se rapporte en outre à l'utilisation d'au moins un antagoniste des canaux calciques pour la préparation d'une composition pharmaceutique destinée à diminuer la lipogénèse sébacée, et, plus particulièrement, à prévenir et/ou traiter l'acné associé à une hyperséborrhée.

La présente invention a enfin pour objet un procédé de traitement cosmétique en vue de prévenir et/ou traiter les peaux grasses comprenant l'administration orale d'une composition comprenant au moins un antagoniste des canaux calciques.

Elle a également pour objet un procédé de traitement cosmétique en vue de prévenir et/ou traiter les peaux grasses comprenant l'application topique sur la peau d'une composition comprenant au moins un antagoniste des canaux calciques.

Les exemples suivants de compositions suivants illustrent l'invention sans la limiter aucunement.

### Exemple 1 - Mesure de l'activité des sels de manganèse

Les sels de manganèse sont évalués dans le modèle de sébocytes humains immortalisés en culture, issus de la lignée SZ95 décrite dans Zouboulis, C.C., Seltmann, H., Neitzel, H. & Orfanos, C.E., Establishment and Characterization of an Immortalized Human Sebaceous Gland Cell Line, J. Invest. Dermatol., 113, 1011-1020 (1999).
Le test a consisté à mesurer la quantité de lipides produite par les sébocytes de la lignée (à confluence), en présence ou non d'agents actifs, dilué dans le DMSO, de telle sorte que la quantité finale de DMSO dans le milieu de culture soit 0,1%. Après 2 jours de traitement, les cellules adhérentes sont traitées par du Rouge de Nile (1□g/ml). Le contenu en lipides est ensuite quantifié par mesure de la fluorescence du colorant (deux couples d'excitation/émission : 485-540nm pour les lipides neutres et 540-620 nm pour les lipides non neutres). Les résultats sont donnés pour les lipides neutres (caractéristiques du sébum).
L'expérience est réalisée en octoplicate et renouvelée 2 fois.

### Exemple 2 - Formulations

### A. Par voie orale

| **Dragées** | |
|---|---|
| ***Matières actives*** | ***mg*****/*****dragée*** |
| sel de manganèse d'acide azélaique | 300 |

| ***Excipient du noyau de la dragée*** | |
|---|---|
| Cellulose mino-cristalline | 70 |
| Encompress™ | 60 |
| Stearate de Magnesium | 3 |
| Silice colloïdale anhydre | 1 |

| ***Agent d'enrobage*** | |
|---|---|
| Gomme laque | 5 |
| Talc | 61 |
| Polyvidone | 6 |

Ce type de dragée peut être pris 1 à 4 fois par jour.

| **Capsules pour une absorption orale** | |
|---|---|
| ***Matières active*** | **mg/capsule** |
| sel de manganèse d'acide séborique | 200 |
| Huile de bourrache | 200 |
| Huile de pépins de cassis | 200 |

| ***Vitamines*** | |
|---|---|
| Vitamine C | 60 |
| Vitamine E | 2 |

### B. Par voie topique

Ces compositions sont obtenues par les techniques habituelles couramment utilisées en cosmétique ou en pharmacie.

### Gel niosomé

- Chimexane NS® 1,80 g
- Stéaroylglutamate monosodique 0,20 g
- sel de manganèse d'acide sébacique 1,00 g
- Carbomer 0,20 g
- Triéthanolamine qs pH = 7
- Conservateurs qs
- Parfums qs
- Eau déminéralisée qsp 100,00 g
On applique ce gel sur la peau, une à deux fois par jour.

### Lotion niosomée

- Chimexane NL® 0,475 g
- Cholestérol 0,475 g
- Stéaroylglutamate monosodique 0,050 g
- oléate de manganèse 2,000 g
- Conservateurs qs
- Colorants qs
- Parfum qs
- Eau déminéralisée qsp 100,000 g
On applique cette lotion sur la peau, une à deux fois par jour.

### Lotion

- citrate de manganèse 2,00 g
- Dowanol PM®** 20,00 g
- Klucel G®* 3,00 g
- Alcool éthylique 40,00 g
- Eau qsp 100,00 g

** : Monométhyléther de propylèneglycol vendu par la société Dow Chemical
* : Hydroxypropylcellulose vendue par la société Hercules

On applique 1 ml de cette lotion sur la peau, à la fréquence de une à deux fois par jour.

| **Lotion pour le cuir chevelu** | |
|---|---|
| sel de manganèse d'acide azélaique | 5,00 % |
| Antioxydant | 3,00 % |
| Isopropanol | 40,00 % |
| Conservateur | 2,00 % |
| Eau | qsp 100,00% |

| **Crème de soin (émulsion huile dans eau)** | |
|---|---|
| Chlorure de manganèse | 5,00 % |
| Stéarate de glycérol | 2,00 % |
| Polysorbate 60 (Tween 60®vendu par ICI) | 1,00 % |
| Acide stéarique | 2,00 % |
| Triéthanolamine | 0,80 % |
| Carbomer | 0,40 % |
| Fraction liquide du beurre de karité | 12,00 % |
| Perhydrosqualène | 12,00 % |
| Antioxydant | 2,00 % |
| Eau | qsp 100,00 % |

## Revendications

1. Utilisation cosmétique d'au moins un antagoniste des canaux calciques choisi parmi les composés suivants : chlorpromazine, verapamil, nifedipine, prenylamine, nisoldipine ou manganèse sous forme ionique ou sous forme de sels minéraux ou organiques isolés et/ou présents dans des extraits naturels, végétaux ou bactériens pour diminuer la lipogénèse sébacée.

2. Utilisation selon la revendication 1, pour prévenir et/ou corriger les altérations cutanées provoquées et/ou associées à une hypersécrétion sébacée.

3. Utilisation selon la revendication 1 ou 2, pour prévenir et/ou traiter les peaux grasses.

4. Utilisation selon l'une quelconque des revendications 1 à 3, **caractérisée en ce que** l'antagoniste des canaux calciques est du manganèse sous forme de sels organiques présents dans des extraits naturels végétaux, tels que des extraits de noix (*Juglans regia*).

5. Utilisation selon la revendication 4, **caractérisée en ce que** l'antagoniste des canaux calciques est du manganèse sous forme de sel de manganèse d'un acide carboxylique tel que le gluconate de manganèse, le carbonate de manganèse, l'acétate de manganèse, le citrate de manganèse, l'oléate de manganèse, l'oxalate de manganèse, le linéolate de manganèse ou le linolénate de manganèse ou de sel de manganèse d'un acide aliphatique dicarboxylique tel que l'acide sébacique, l'acide séborique ou l'acide azélaique.

6. Utilisation selon la revendication 4, **caractérisée en ce que** l'antagoniste des canaux calciques est du manganèse sous forme de sels minéraux tels que le chlorure de manganèse, le borate de manganèse, le nitrate de manganèse, le phosphate de manganèse ou le sulfate de manganèse.

7. Utilisation selon l'une quelconque des revendications 1 à 6, **caractérisée en ce que** l'antagoniste des canaux calciques est formulé dans une composition destinée à être administrée par voie orale.

8. Utilisation selon la revendication 7, **caractérisée en ce que** la composition se présente sous forme de complément nutritionnel comprenant un extrait de graines de Juglandacée et/ou du manganèse sous forme d'oligo-élément.

9. Utilisation selon la revendication 7 ou 8, **caractérisée en ce que** l'antagoniste des canaux calciques est présent à une concentration comprise entre 10 et 1000 mg poids / kg de poids corporel / jour.

10. Utilisation selon l'une quelconque des revendications 7 à 9, **caractérisée en ce que** la composition comprend un actif complémentaire choisi parmi : les rétinoïdes, les caroténoïdes, la vitamine C et les agents antibactériens.

11. Utilisation selon l'une quelconque des revendications 1 à 6, **caractérisée en ce que** l'antagoniste des canaux calciques est formulé dans une composition destinée à être appliquée par voie topique.

12. Utilisation selon la revendication 11, **caractérisée en ce que** l'antagoniste des canaux calciques est un sel de manganèse d'un acide aliphatique dicarboxylique, de préférence l'acide sébacique, l'acide séborique ou l'acide azélaique.

13. Utilisation selon la revendication 11 ou 12, **caractérisée en ce que** l'antagoniste des canaux calciques est présent à une concentration comprise entre 0,05 à 40 % du poids total de la composition.

14. Utilisation selon l'une quelconque des revendications 11 à 13, **caractérisée en ce que** la composition comprend un actif complémentaire choisi parmi : d'autres agents antiséborrhéiques, des agents antimicrobiens et les phytoaléxines, des agents antiseptiques, des agents anti-inflammatoires et/ou anti-irritants et des agents kératolytiques.

15. Utilisation d'au moins un antagoniste des canaux calciques pour la préparation d'une composition pharmaceutique destinée à diminuer la lipogénèse sébacée.

16. Utilisation selon la revendication 15, **caractérisée en ce que** la composition pharmaceutique est destinée à prévenir et/ou traiter l'acné associé à une hyperséborrhée.

17. Procédé de traitement cosmétique en vue de prévenir et/ou traiter les peaux grasses comprenant l'administration orale d'une composition comprenant au moins un antagoniste des canaux calciques.

18. Procédé de traitement cosmétique en vue de prévenir et/ou traiter les peaux grasses comprenant l'application topique sur la peau d'une composition comprenant au moins un antagoniste des canaux calciques.
